# EUROPÄISCHE PATENTANMELDUNG

(11) **EP 4 576 105 A1**
(43) Veröffentlichungstag der Anmeldung: **25.06.2025**
(21) Anmeldenummer: 23217665.1
(22) Anmeldetag: 18.12.2023
(51) Int. Cl.: G16H 10/20, G16H 10/60, G16H 40/67, G06Q 10/00, G06F 21/62, H04L 9/40, H04L 67/00

(54) **DATENSCHUTZWAHRENDES ANALYSESYSTEM UND ANALYSEVERFAHREN FÜR FÖDERIERTE PATIENTENDATEN**

(71) Anmelder: Clinerion Ltd., 4051 Basel (CH)
(72) Erfinder: Berchier, Matteo, 7742 Poschiavo (CH); Walter, Andreas, 76532 Baden-Baden (DE); Bodenmann, Bernhard, 4102 Binningen (CH)
(74) Vertreter: Daub, Thomas

(57) **Zusammenfassung**

Es wird ein datenschutzwahrendes Analysesystem (36) für föderierte Patientendaten, mit zumindest einer Mehrzahl an voneinander räumlich und systemtechnisch getrennten Analyseumgebungen (10, 10'), welche jeweils zumindest eine lokale Patientenanalysedatenbank (12) mit einem Teil der föderierten Patientendaten umfassen, und welche jeweils zumindest ein lokales Analysemodul (14) umfassen, das zumindest zu einer Ausführung von Skripten auf den in der jeweiligen lokalen Patientenanalysedatenbank (12) verfügbaren Teil der föderierten Patientendaten vorgesehen ist,
und mit zumindest einem, insbesondere zentralen oder verteilt angeordneten, externen Anwender- und/oder Programmier-Schnittstellensystem (16), welches räumlich und systemtechnisch von den Analyseumgebungen (10, 10') getrennt ist, über welches die von den lokalen Analysemodulen (14) ausführbaren Skripte, insbesondere extern, erstellbar und/oder bereitstellbar sind und welches mit jeder der Analyseumgebungen (10, 10') datentransfertechnisch ausschließlich über von den Analyseumgebungen (10, 10') ausgehende Verbindungen, insbesondere Outbound-Verbindungen, verbunden ist, vorgeschlagen.

## Beschreibung

### Stand der Technik

Die Erfindung betrifft ein Analysesystem nach dem Anspruch 1, eine Analyseumgebung oder ein Schnittstellensystem nach dem Anspruch 15 und ein Analyseverfahren nach dem Anspruch 16.

Es ist bereits vorgeschlagen worden Patientendaten von Spitalinformationssystemen für vielfältige Zwecke zu analysieren, z.B. um Probanden für klinische Studien zu identifizieren oder um Behandlungserfolge zu analysieren, etc. Da es sich bei den Daten, die in Spitalinformationssystemen abgelegt sind, um besonders sensible und schützenswerte Daten handelt, ist die Datensicherheit von entscheidender Bedeutung.

Die Aufgabe der Erfindung besteht insbesondere darin, eine gattungsgemäße Vorrichtung mit vorteilhaften Eigenschaften hinsichtlich einer datenschutzwahrenden Analyse von Patientendaten bereitzustellen. Die Aufgabe wird erfindungsgemäß durch die Merkmale der unabhängigen Patentansprüche gelöst, während vorteilhafte Ausgestaltungen und Weiterbildungen der Erfindung den Unteransprüchen entnommen werden können.

### Vorteile der Erfindung

Es wird ein datenschutzwahrendes Analysesystem für föderierte Patientendaten, insbesondere zu einer Rekrutierung von Probanden für klinische Studien, zu einer Ermittlung von Trends, zur einer Erlangung von Optimierungsvorschlägen für Behandlungen, zu einer Umsetzung von klinischen Dashboards und/oder zur Modellierung von Verfahren des maschinellen Lernens, mit zumindest einer Mehrzahl an voneinander räumlich und systemtechnisch getrennten Analyseumgebungen, welche jeweils zumindest eine lokale Patientenanalysedatenbank mit einem Teil der föderierten Patientendaten umfassen, und welche jeweils zumindest ein lokales Analysemodul umfassen, das zumindest zu einer Ausführung von Skripten auf den in der jeweiligen lokalen Patientenanalysedatenbank verfügbaren Teil der föderierten Patientendaten vorgesehen ist, und mit zumindest einem, insbesondere zentralen oder verteilt angeordneten, externen Anwender- und/oder Programmier-Schnittstellensystem, welches räumlich und systemtechnisch von den Analyseumgebungen getrennt ist, über welches die von den lokalen Analysemodulen ausführbaren Skripte, insbesondere extern, erstellbar und/oder bereitstellbar sind und welches mit jeder der Analyseumgebungen datentransfertechnisch ausschließlich über von den Analyseumgebungen ausgehende Verbindungen, insbesondere sogenannte Outbound-Verbindungen, verbunden ist, vorgeschlagen. Dadurch kann vorteilhaft eine auf der Ausführung von Skripten basierende datenschutzwahrende Analysemöglichkeit bereitgestellt werden. Vorteilhaft kann eine besonders vielfältig verwendbare Analysemöglichkeit geschaffen werden, welche zugleich einen besonders hohen Grad an Datenschutz für sensible Patientendaten besitzt. Vorteilhaft kann eine Möglichkeit zu einer lokalen Ausführung von extern erstellten Skripten zur Datenanalyse bereitgestellt werden, welche lediglich auf Outbound-Verbindungen basiert und somit keine externen Zugriffsmöglichkeiten auf die lokalen Patientenanalysedatenbanken öffnet. Dadurch kann vorteilhaft erreicht werden, dass eine Installation des Analysesystems die Datensicherheit der die lokalen Patientenanalysedatenbanken umfassenden Spitalinformationssysteme in keiner Weise negativ beeinflussen kann. Das vorgeschlagene Analysesystem stellt vorteilhaft die Möglichkeit bereit, extern vorbereitete Skripte auf nur lokal verfügbare und besonders sensible Patientendaten aus Spitalinformationssystemen anzuwenden, ohne dabei Datenlecks erzeugen zu können, durch die sensible Informationen die lokalen Spitalinformationssysteme verlassen könnten. Vorteilhaft erzeugt das vorgeschlagene Analysesystem durch die vorgeschlagene Systemarchitektur keine Exploit- oder Hackmöglichkeiten, durch welche ein unbefugter Zugriff auf die lokalen Systeme, wie die Spitalinformationssysteme, möglich werden könnte. Insbesondere sind zu einer Maximierung einer Sicherheit des Analysesystems alle Skripte des Analysesystems, die von den lokalen Analysemodulen ausführbar sein sollen einer manuellen oder automatisierten Sicherheitsüberprüfung / einem manuellen oder automatisierten Skriptencheck unterworfen. Vorteilhaft kann dadurch ein durch die Skripten entstehendes Sicherheitsleck ausgeschlossen werden. Vorteilhaft kann eine besonders hohe Datensicherheit erreicht werden. Vorteilhaft kann ein auf Skripten basierendes Analysesystem mit einer besonders datensicheren Systemarchitektur bereitgestellt werden.

Darunter, dass das Analysesystem "datenschutzwahrend" ist soll insbesondere verstanden werden, dass das Analysesystem keine (beabsichtigten und unbeabsichtigten) Wege bietet, über die sensible Daten, z.B. Patientendaten, oder andere Daten, aus denen irgendwelche Rückschlüsse auf Einzelpersonen gezogen werden könnten, einen geschützten Bereich (die Analyseumgebung bzw. das Spitalinformationssystem), mit dem das Analysesystem kommuniziert, verlassen könnten. Föderierte Patientendaten sind insbesondere föderiert vorgehaltene, vorzugsweise an unterschiedlichen Orten von unterschiedlichen Analyseumgebungen vorgehaltene Patientendaten. Föderierte Patientendaten sind insbesondere in föderierten Patientendatenbanken (den einzelnen Patientendatenbanken der einzelnen Analyseumgebungen) abgespeichert und können von dem Analysesystem nicht in diesen Patientendatenbanken verändert werden. Insbesondere verlassen weder die föderierten Patientendaten, noch Teile der föderierten Patientendaten oder Kopien davon die lokalen Analyseumgebungen, vorzugsweise die Spitalinformationssysteme. Vorzugsweise können über das Analysesystem lediglich aggregierte Daten / Reporte / Ergebnisse, wie z.B. Statistiken, die lokalen Analyseumgebungen, vorzugsweise die Spitalinformationssysteme, verlassen. Die lokalen Analyseumgebungen können Gesundheitsinformationssysteme / Spitalinformationssysteme (HIS) oder Teile von Gesundheitsinformationssystemen / Spitalinformationssystemen unterschiedlicher Gesundheitsinstitutionen / Krankenhäuser sein. Diese Gesundheitsinformationssysteme / Spitalinformationssysteme sind in der Regel jeweils einer Gesundheitsinstitution / einem Krankenhaus fest zugeordnet und nach außen gegen unbefugten Zugriff gesichert. Die Analyseumgebungen können als Teil eines Intranets, insbesondere der Gesundheitsinstitutionen / Krankenhäuser, ausgebildet sein oder vorzugsweise in Intranets, insbesondere der Gesundheitsinstitutionen / Krankenhäuser, angeordnet sein. Vorzugsweise sind diese Intranets gegen Zugriff von außen, z.B. aus dem Internet, geschützt, beispielsweise durch eine physische Trennung vom Internet oder durch datensicherheitstechnische Maßnahmen, wie Firewalls oder dergleichen. Bevorzugt ist jede der lokal in den Intranets angeordneten Analyseumgebungen als ein software- und/oder hardwaretechnisches Modul ausgebildet, welches in dem jeweiligen Gesundheitsinformationssystem / Spitalinformationssystem installiert wurde. Es ist beispielsweise denkbar, dass die jeweilige Analyseumgebung als ein separater Server ausgebildet ist, welcher in das jeweilige Intranet integriert ist oder dass die jeweilige Analyseumgebung als ein auf einen existierenden Server des jeweiligen Intranets aufgespieltes Softwaremodul ausgebildet ist. Ein möglicher Weg der Installation der Analyseumgebung ist eine Installation auf einem Server, der in der jeweiligen Gesundheitsinstitution / in dem jeweiligen Krankenhaus bereitgestellt wird.

Unter einer "räumlichen Trennung" soll insbesondere eine geographische Trennung verstanden werden, welche bevorzugt zumindest mehrere Kilometer beträgt. Insbesondere ist unter der räumlichen Trennung eine Anordnung in mehreren verschiedenen Krankenhäusern / Krankenhaus-Intranets zu verstehen. Unter einer "systemtechnischen Trennung" ist insbesondere eine Anordnung in / Zuordnung zu unterschiedlichen und vorzugsweise miteinander nicht verbundenen Gesundheitsinformationssystemen / Spitalinformationssystemen, vorzugsweise deren Intranets, zu verstehen. Insbesondere umfasst jede lokale

Patientenanalysedatenbank lediglich einen Teil der föderierten Patientendaten, mit denen das Analysesystem arbeitet. Dabei sind die lokalen Patientenanalysedatenbanken vorzugsweise zumindest im Wesentlichen überlappungsfrei. In Einzelfällen kann es jedoch zu Überlappungen kommen, beispielsweise wenn Patienten in zwei Krankenhäusern gleichzeitig in Behandlung sind. Die lokale Patientenanalysedatenbank umfasst vorzugsweise lediglich anonymisierte und/oder de-identifizierte Patientendaten. Die in der lokalen Patientenanalysedatenbank umfassten anonymisierten und/oder de-identifizierten Patientendaten werden aus originalen föderierten Patientendaten erstellt, welche in den Spitalinformationssystemen / Gesundheitsinformationssystemen abgespeichert sind. Beispielsweise bezieht die jeweilige lokale Analyseumgebung, vorzugsweise ein lokales Datenextraktionsmodul der lokalen Analyseumgebung, die originalen föderierten Patientendaten von den Patientenakten, die in demselben Spitalinformationssystem / Gesundheitsinformationssystem angeordnet sind wie die jeweilige Analyseumgebung, anonymisiert und/oder deidentifiziert diese (z.B. mittels eines Anonymisierungsmoduls der jeweiligen Analyseumgebung) und speichert diese dann in die lokale Patientenanalysedatenbank der jeweiligen Analyseumgebung so ab, dass sie für Analysen des lokalen Analysemoduls bereitstehen. Das jeweilige lokale Analysemodul ist vorzugsweise zumindest teilweise in die jeweilige Analyseumgebung integriert ausgebildet. Insbesondere kann das jeweilige lokale Analysemodul zumindest teilweise Hardware eines Computersystems der jeweiligen Analyseumgebung, z.B. einen Prozessor oder einen Speicher, verwenden und/oder mit anderen Modulen der jeweiligen Analyseumgebung teilen. Alternativ kann das jeweilige lokale Analysemodul auch zumindest teilweise als separater Server innerhalb des Computersystems der Analyseumgebung und/oder innerhalb des Intranets des jeweiligen Spitalinformationssystems implementiert sein. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Es ist denkbar, dass mehrere Intranets zu einem cloudbasierten Extranet im gleichen Land, wie dies beispielsweise im brasilianischen Gesundheitssystem umgesetzt ist, zusammengeschlossen sind.

Ein Skript soll insbesondere als ein Textdokument mit Befehlen, die ein Computer verstehen kann, verstanden werden. Beispielsweise kann das Skript, insbesondere ein Quellcode des Skripts, in der Programmiersprache Python, in der Programmiersprache R, in der Programmiersprache Java oder in einer anderen Programmiersprache verfasst sein. Skripte sollen insbesondere als Programmanweisungen zur Ausführung auf die Patientendaten, insbesondere die jeweils lokal verfügbaren Teile der föderierten Patientendaten, verstanden werden. Einfache Skripte können beispielsweise eine Häufigkeit bestimmter Patientenfakten innerhalb der jeweils lokal verfügbaren Patientendaten zählen. Umfangreichere Skripte können beispielsweise Beziehungen zwischen Fakten (z.B. ein Wechsel von einer First Line Behandlung zu einer Second Line Behandlung) aus den jeweils lokal verfügbaren Patientendaten ermitteln. Umfangreichere Skripte können beispielsweise Muster oder Behandlungs- / Diagnoseabfolgen herausfiltern (z.B. "erst Medikament 1, dann Medikament 2" oder erst "Diagnose X, dann Behandlung Y", etc.). Komplexe Skripte können beispielsweise Anweisungen zur (lokalen) Erstellung von Modellen für maschinelles Lernen, welche vorzugsweise auf die jeweils lokal verfügbaren Patientendaten angewendet werden sollen, enthalten.

Das externe Anwender- und/oder Programmier-Schnittstellensystem ist vorzugsweise als ein zentraler dedizierter Server ausgebildet, welcher eine Zugriffsmöglichkeit für Nutzer (z.B. Anwender und/oder Skripten-Programmierer) des Analysesystems, z.B. Wissenschaftler, Pharmaunternehmen, etc., bietet. Alternativ kann das externe Anwender- und/oder Programmier-Schnittstellensystem auch als verteiltes System, z.B. als Cloud-Computing-System ausgebildet sein. Vorzugsweise ist das externe Anwender- und/oder Programmier-Schnittstellensystem außerhalb jeglicher Spitalinformationssysteme / Gesundheitsinformationssysteme und/oder deren Intranets angeordnet. Insbesondere stellt das externe Anwender- und/oder Programmier-Schnittstellensystem ein Nutzerinterface zu einem Erstellen von Skripten und/oder zu einem Hochladen von bereits erstellten Skripten bereit. Insbesondere ist das externe Anwender- und/oder Programmier-Schnittstellensystem dazu vorgesehen, die Skripten für ein Abholen durch die Analyseumgebung(en) bereitzustellen. Insbesondere ist das externe Anwender- und/oder Programmier-Schnittstellensystem nicht dazu in der Lage, die Skripten unaufgefordert oder auf Eigeninitiative bzw. Nutzerbefehl an die Analyseumgebung(en) zu senden. Insbesondere erlauben die Analyseumgebungen keinen Aufbau einer von dem externen Anwender- und/oder Programmier-Schnittstellensystem ausgehenden Inbound-Verbindung. Insbesondere blocken die Analyseumgebungen jegliche Versuche des externen Anwender- und/oder Programmier-Schnittstellensystems Inbound-Verbindung mit den Analyseumgebungen, die von dem externen Anwender- und/oder Programmier-Schnittstellensystem initiiert sind, aufzubauen.

Insbesondere sind die Analyseumgebungen frei von Inbound Ports und weisen ausschließlich Outbound Ports auf, wodurch vorteilhaft sichergestellt werden kann, dass Verbindungen zwischen dem externen Anwender- und/oder Programmier-Schnittstellensystem und den Analyseumgebungen immer nur von der Seite der Analyseumgebungen initiiert werden können. Dadurch kann vorteilhaft erreicht werden, dass der komplette Datentransfer von dem externen Anwender- und/oder Programmier-Schnittstellensystem für die Seite der Analyseumgebungen vollständig transparent ist. Zudem kann vorteilhaft ein unautorisierter Zugriff auf die Analyseumgebungen über das externe Anwender- und/oder Programmier-Schnittstellensystem, beispielsweise durch "hacking", verunmöglicht werden. Unter einer "Outbound-Verbindung" soll insbesondere eine Verbindung zwischen zumindest einem Teil zumindest einer der Analyseumgebungen und dem externen Anwender- und/oder Programmier-Schnittstellensystem verstanden werden, welche ausschließlich von einer Seite der Analyseumgebungen initiierbar ist. Insbesondere ist ein Datentransport zwischen zumindest einem Teil zumindest einer der Analyseumgebungen und dem externen Anwender- und/oder Programmier-Schnittstellensystem mittels einer Outbound-Verbindung ausschließlich von der Seite der Analyseumgebungen steuerbar. Insbesondere sind Datentransporteinheiten der Analyseumgebungen frei von offenen Ports. Insbesondere kann das externe Anwender- und/oder Programmier-Schnittstellensystem ausschließlich Daten an die Analyseumgebungen senden, welche von zumindest einer der Analyseumgebungen zuvor angefordert wurden. Vorzugsweise ist der Datentransfer zwischen den Analyseumgebungen und dem externen Anwender- und/oder Programmier-Schnittstellensystem frei von unanonymisierten Datensätzen, insbesondere unanonymisierten Patientendatensätzen. Vorzugsweise ist der Datentransfer zwischen den Analyseumgebungen und dem externen Anwender- und/oder Programmier-Schnittstellensystem frei von ursprünglich individuelle Personen beschreibenden unaggregierten anonymisierten Datensätzen, insbesondere anonymisierten Patientendatensätzen. Vorzugsweise ist der Datentransfer zwischen den Analyseumgebungen und dem externen Anwender- und/oder Programmier-Schnittstellensystem frei von Identifikatoren und/oder Merkmalen, aus welchen, insbesondere unter Einhaltung von Datenschutzverordnungen und -gesetzen, eine Person und/oder ein Patient identifizierbar sein könnte. Insbesondere ist der Datentransfer von den Analyseumgebungen zu dem externen Anwender- und/oder Programmier-Schnittstellensystem auf aggregierte Daten / Reporte / Ergebnisse, welche frei sind von individuellen Personen zuordenbaren anonymisierten oder unanonymisierten Patientendaten, beschränkt.

Insbesondere sind die Analyseumgebungen dazu vorgesehen, deren Analyseergebnisse lediglich als aggregierte Daten / als ein aggregierter Report / als aggregierte Ergebnisse (z.B. Trefferanzahlen und/oder Trefferkategorien) an das externe Anwender- und/oder Programmier-Schnittstellensystem zu senden / dem externen Anwender- und/oder Programmier-Schnittstellensystem zu Verfügung zu stellen. Insbesondere sind die Analyseumgebungen dazu vorgesehen, deren ermittelte Analyseergebnisse lediglich in Form von Daten zu versenden, anhand welcher überhaupt keine Informationen entnehmbar sind, mittels denen eine Person und/oder ein Patient identifizierbar sein könnte.

Ferner wird vorgeschlagen, dass zumindest ein Großteil der Analyseumgebungen, insbesondere zumindest alle innerhalb zumindest eines Landes, beispielsweise der USA, Deutschlands, Frankreichs oder der Schweiz, etc., angeordnete Analyseumgebungen, vorzugsweise alle existierenden Analyseumgebungen, innerhalb von speziell gesicherten und/oder zugangsbeschränkten Bereichen, z.B. Intranets, von Gesundheitsinformationssystemen, insbesondere Spitalinformationssystemen, angeordnet ist/sind und dass das Anwender- und/oder Programmier-Schnittstellensystem außerhalb dieser speziell gesicherten und/oder zugangsbeschränkten Bereiche von Gesundheitsinformationssystemen, insbesondere Spitalinformationssystemen, angeordnet ist. Dadurch kann vorteilhaft eine hohe Datensicherheit bei gleichzeitiger Bereitstellung einer zentralen Durchsuchbarkeit erreicht werden. Insbesondere kann eine besonders vorteilhafte Systemarchitektur für Analysesysteme bereitgestellt werden, welche eine hohe Datensicherheit und eine hohe Benutzerfreundlichkeit kombiniert. Insbesondere kann eine besonders vorteilhafte Systemarchitektur für Analysesysteme bereitgestellt werden, welche eine (zentrale) Verwendung von Skripten zur Datenanalyse erlaubt und zugleich eine maximale Datensicherheit wahrt. Unter einem Großteil soll insbesondere 85 %, vorzugsweise 90 %, bevorzugt 95 % und besonders bevorzugt 99 % verstanden werden. Besonders vorteilhaft sind alle Analyseumgebungen innerhalb von speziell gesicherten und/oder zugangsbeschränkten Bereichen, z.B. Intranets, von Gesundheitsinformationssystemen, insbesondere Spitalinformationssystemen, angeordnet, eine Anordnung einzelner Analyseumgebungen, die als überwiegendes Ziel die Umgehung eines Schutzbereichs eines Patents hat und keine wesentlichen technischen Vorteile mit sich bringt, soll nicht als Abweichung von dem Begriff "alle" verstanden werden.

Des Weiteren wird vorgeschlagen, dass die Analyseumgebungen jeweils zumindest ein Skript-Abholmodul umfassen, welches dazu vorgesehen ist, eine, insbesondere regelmäßige, Abfrage des externen Schnittstellensystems nach neuen, von dem externen Anwender- und/oder Programmier-Schnittstellensystem für die lokalen Analysemodule bereitgestellten, Skripten durchzuführen. Dadurch kann vorteilhaft eine besonders hohe Systemsicherheit, insbesondere eine besonders sichere Systemarchitektur, bereitgestellt werden. Vorteilhaft können lediglich Skripte in die Analyseumgebungen gelangen, welche von den Skript-Abholmodulen der Analyseumgebungen gezielt und gewollt abgeholt wurden. Vorteilhaft kann verhindert werden, dass unerwünschte / maliziöse Skripte auf die Analyseumgebungen gelangen können. Insbesondere schaut das Skript-Abholmodul nur an einer oder mehreren bekannten und/oder vertrauenswürdigen Stellen nach neuen Skripten für den Download durch die jeweilige Analyseumgebung. Insbesondere können die Skripte für eine Auswahl aller Analyseumgebungen oder für alle Analyseumgebungen zum Abholen bereitgestellt werden. Die Auswahl der Analyseumgebungen dafür kann beispielsweise durch Metadaten oder durch die Auswahl bestimmter Bereitstellungsorte / -pfade ("Stellen"), an denen nur bestimmte Analyseumgebungen suchen, mitgeteilt werden. Bevorzugt ist jedes der Skript-Abholmodule als ein software- und/oder hardwaretechnisches Modul ausgebildet, welches in dem jeweiligen Gesundheitsinformationssystem / Spitalinformationssystem installiert wurde. Das jeweilige Skript-Abholmodul ist vorzugsweise zumindest teilweise in die jeweilige Analyseumgebung integriert ausgebildet. Insbesondere kann das jeweilige Skript-Abholmodul zumindest teilweise eine Hardware eines Computersystems der jeweiligen Analyseumgebung, z.B. einen Prozessor oder einen Speicher, verwenden und/oder mit anderen Modulen der jeweiligen Analyseumgebung teilen. Alternativ kann das jeweilige Skript-Abholmodul auch zumindest teilweise als separater Server innerhalb des Computersystems der Analyseumgebung und/oder innerhalb des Intranets des jeweiligen Spitalinformationssystems implementiert sein. Insbesondere erfolgt die Abfrage durch das Skript-Abholmodul unaufgefordert und vorzugsweise unbeeinflusst von außerhalb, insbesondere unbeeinflusst durch das externe Anwender- und/oder Programmier-Schnittstellensystem. Die Abfrage kann beispielsweise periodisch, z.B. jede Minute, jede Stunde, jeden Tag, etc., erfolgen. Die Abfrageperiode ist dabei vorzugsweise konfigurierbar. Es ist jedoch auch denkbar, dass von der Seite eines oder mehrerer Analyseumgebungen eine Abfrage manuell erzwungen werden kann. Es ist denkbar, dass das Skript-Abholmodul immer alle bereitgestellten Skripte abholt oder vor einem Download einen Vergleich vornimmt und nur die Skripte abholt, die bisher noch nicht auf der Analyseumgebung vorhanden waren. Bevorzugt lädt das Skript-Abholmodul immer nur die neuen, bisher noch nicht auf der Analyseumgebung vorhandenen Skripte herunter. Wenn die Abfrage ergibt, dass abzuholende neue Skripte bereitgestellt sind, erfolgt der Download der Skripte auf die Analyseumgebung(en) mittels der von den jeweiligen Analyseumgebungen initiierten Verbindungen, vorzugsweise basierend auf Outbound-Verbindungen.

Wenn dann das Skript-Abholmodul dazu vorgesehen ist, bei einem Auffinden eines neu bereitgestellten Skripts mittels der von den Analyseumgebungen ausgehenden Verbindungen, insbesondere der Outbound-Verbindung, einen Download des bereitgestellten Skripts von dem externen Schnittstellensystem zu veranlassen, kann vorteilhaft eine besonders hohe Systemsicherheit, insbesondere eine besonders sichere Systemarchitektur, bereitgestellt werden. Vorteilhaft können lediglich Skripte in die Analyseumgebungen gelangen, welche von den Skript-Abholmodulen der Analyseumgebungen gezielt und gewollt abgeholt wurden. Vorteilhaft kann verhindert werden, dass unerwünschte / maliziöse Skripte auf die Analyseumgebungen gelangen können.

Weiterhin wird vorgeschlagen, dass die Analyseumgebung eine lokale Programmbibliothek-Datenbank aufweist, welche Programmbibliotheken, z.B. Python-Programmbibliotheken oder Programmbibliotheken anderer Programmiersprachen, und/oder Software-Hilfsmodule umfasst, die von den Skripten aufrufbar sind, und dass in der Analyseumgebung ein durch die Skripte initiiertes Aufrufen und/oder Herunterladen von externen Programmbibliotheken und/oder Software-Hilfsmodulen, die nicht in der lokalen Programmbibliothek-Datenbank umfasst sind, z.B. über das Internet, unterbunden ist. Dadurch kann vorteilhaft eine besonders hohe Systemsicherheit, insbesondere eine besonders sichere Systemarchitektur, bereitgestellt werden. Vorteilhaft können lediglich Programmbibliotheken und/oder Software-Hilfsmodule in der Analyseumgebung, z.B. durch das lokale Analysemodul, ausgeführt werden, welche zuvor einer Sicherheitsprüfung unterworfen werden können. Vorteilhaft kann eine vollständige Kontrolle und/oder Transparenz über alle innerhalb der Analyseumgebung ausgeführte Programmbibliotheken und/oder Software-Hilfsmodule erreicht werden. Vorteilhaft kann verhindert werden, dass unerwünschte / maliziöse Programmbibliotheken und/oder Software-Hilfsmodule auf die Analyseumgebungen gelangen können und/oder von der Analyseumgebung / dem lokalen Analysemodul ausgeführt werden können. Insbesondere ist jeder der Programmbibliotheken der lokalen Programmbibliotheken-Datenbanken eine Versionsnummer zugeordnet. Insbesondere wird jede der Programmbibliotheken lokal mit der zugehörigen Versionsnummer installiert. Vorzugsweise wird zu einer Aktualisierung einer Programmbibliothek die Versionsnummer abgeglichen. Skripte, die auf eine Programmbibliothek zugreifen, müssen vorzugsweise kompatibel zu der aktuell installierten / gültigen Versionsnummer der Programmbibliothek sein.

Wenn nun die Analyseumgebungen jeweils zumindest ein Bibliothek-Abholmodul umfassen, welches dazu vorgesehen ist, eine, insbesondere regelmäßige, Abfrage des externen Anwender- und/oder Programmier-Schnittstellensystems nach neuen, von dem externen Anwender- und/oder Programmier-Schnittstellensystem für die lokale Programmbibliothek-Datenbank bereitgestellten, insbesondere autorisierten, externen Programmbibliotheken und/oder Software-Hilfsmodulen durchzuführen und insbesondere bei einem Auffinden einer/eines neu bereitgestellten insbesondere autorisierten, externen Programmbibliothek und/oder Software-Hilfsmoduls mittels der von den Analyseumgebungen ausgehenden Verbindungen, vorzugsweise der Outbound-Verbindungen, einen Download der/des bereitgestellten Programmbibliothek und/oder Software-Hilfsmoduls, vorzugsweise von dem externen Anwender- und/oder Programmier-Schnittstellensystem, zu veranlassen, kann vorteilhaft eine besonders hohe Systemsicherheit, insbesondere eine besonders sichere Systemarchitektur, bereitgestellt werden. Vorteilhaft kann eine vollständige Kontrolle und/oder Transparenz über alle innerhalb der Analyseumgebung ausgeführte Programmbibliotheken und/oder Software-Hilfsmodule erreicht werden. Insbesondere schaut das Bibliothek-Abholmodul nur an einer oder mehreren bekannten und/oder vertrauenswürdigen Stellen nach neuen Programmbibliotheken und/oder Software-Hilfsmodulen für den Download durch die jeweilige Analyseumgebung. Insbesondere können die Programmbibliotheken und/oder Software-Hilfsmodule für eine Auswahl aller Analyseumgebungen oder für alle Analyseumgebungen zum Abholen bereitgestellt werden. Die Auswahl der Analyseumgebungen dafür kann beispielsweise durch Metadaten oder durch die Auswahl bestimmter Bereitstellungsorte / -pfade ("Stellen"), an denen nur bestimmte Analyseumgebungen suchen, mitgeteilt werden. Bevorzugt ist jedes der Bibliothek-Abholmodule als ein software- und/oder hardwaretechnisches Modul ausgebildet, welches in dem jeweiligen Gesundheitsinformationssystem / Spitalinformationssystem installiert wurde. Das jeweilige Bibliothek-Abholmodul ist vorzugsweise zumindest teilweise in die jeweilige Analyseumgebung integriert ausgebildet. Insbesondere kann das jeweilige Bibliothek-Abholmodul zumindest teilweise Hardware eines Computersystems der jeweiligen Analyseumgebung, z.B. einen Prozessor oder einen Speicher, verwenden und/oder mit anderen Modulen der jeweiligen Analyseumgebung teilen. Alternativ kann das jeweilige Bibliothek-Abholmodul auch zumindest teilweise als separater Server innerhalb des Computersystems der Analyseumgebung und/oder innerhalb des Intranets des jeweiligen Spitalinformationssystems implementiert sein. Insbesondere erfolgt die Abfrage durch das Bibliothek-Abholmodul unaufgefordert und vorzugsweise unbeeinflusst von außerhalb, insbesondere unbeeinflusst durch das externe Anwender- und/oder Programmier-Schnittstellensystem. Die Abfrage kann beispielsweise periodisch, z.B. jede Minute, jede Stunde, jeden Tag, etc., erfolgen. Es ist jedoch auch denkbar, dass von der Seite eines oder mehrerer Analyseumgebungen eine Abfrage manuell erzwungen werden kann oder dass eine Abfrage durch ein Ausführen eines Skripts, welches auf eine noch nicht lokal vorhandene Programmbibliothek und/oder ein noch nicht lokal vorhandenes Software-Hilfsmodul zugreifen möchte, getriggert wird. Es ist denkbar, dass das Bibliothek-Abholmodul immer alle bereitgestellten Programmbibliotheken und/oder Software-Hilfsmodule abholt oder vor einem Download einen Vergleich vornimmt und nur die Programmbibliotheken und/oder Software-Hilfsmodule abholt, die bisher noch nicht auf der Analyseumgebung vorhanden waren oder die aktuell von einem Skript angefordert werden. Wenn die Abfrage ergibt, dass abzuholende neue Programmbibliotheken und/oder Software-Hilfsmodule bereitgestellt sind, erfolgt der Download der Programmbibliotheken und/oder Software-Hilfsmodule auf die Analyseumgebung(en) mittels der von den jeweiligen Analyseumgebungen initiierten Verbindungen, vorzugsweise Outbound Verbindungen. Es ist denkbar, dass die Software-Hilfsmodule und/oder Programmbibliotheken, welche an den Abfragestellen bereitgestellt sind, vorab einer Prüfung, insbesondere einer Sicherheitsüberprüfung unterworfen sind. Vorzugsweise werden lediglich Software-Hilfsmodule und/oder Programmbibliotheken zum Auffinden für die Analyseumgebungen bereitgestellt, welche zuvor eine Sicherheitsüberprüfung bestanden haben. Die Sicherheitsüberprüfung kann automatisiert durch das externe Anwender- und/oder Programmier-Schnittstellensystem durchgeführt werden oder manuell durchgeführt werden. Vorzugsweise werden lediglich explizit freigegebene Software-Hilfsmodule und/oder Programmbibliotheken durch das externe Anwender- und/oder Programmier-Schnittstellensystem für das Auffinden mittels der Bibliothek-Abholmodule bereitgestellt. Insbesondere weisen die Analyseumgebungen Vorkehrungen auf, die eine Installation einer unautorisierten Programmbibliothek oder eines unautorisierten Software-Hilfsmoduls in den Analyseumgebungen verunmöglichen.

Außerdem wird vorgeschlagen, dass das externe Anwender- und/oder Programmier-Schnittstellensystem eine Skript-Testumgebung aufweist, welche dazu vorgesehen ist, neue Skripte oder sich in der Entwicklung befindliche Skripte vor einer Bereitstellung zur Übergabe an die Analyseumgebung/en, insbesondere anhand von durch das externe Anwender- und/oder Programmier-Schnittstellensystem bereitgestellten künstlichen Testumgebungs-Patientendaten, zu testen. Dadurch kann vorteilhaft eine hohe Benutzerfreundlichkeit erreicht werden. Vorteilhaft kann eine hohe Analysequalität, insbesondere eine hohe Qualität der Ergebnisse des Analysesystems, erreicht werden. Die Skript-Testumgebung ist insbesondere über ein von dem externen Anwender- und/oder Programmier-Schnittstellensystem bereitgestelltes Benutzerinterface für externe (außerhalb der Intranets verortete) Nutzer zugänglich. Das externe Anwender- und/oder Programmier-Schnittstellensystem hat Zugriff auf die künstlichen Testumgebungs-Patientendaten, welche keinen realen Personen / Patienten zuordenbar sind. Die künstlichen Testumgebungs-Patientendaten können daher auf dem externen Anwender- und/oder Programmier-Schnittstellensystem abgespeichert sein.

Zudem wird vorgeschlagen, dass die von dem lokalen Analysemodul ausführbaren Skripte, Analyse-Skripte zur rein algorithmischen, insbesondere ML-unabhängigen, Auswertung der Teile der föderierten Patientendaten, auf die das lokale Analysemodul Zugriff hat, sein können (vgl. u.a. die oben erwähnten einfachen und umfangreicheren Skripte). Dadurch kann vorteilhaft ein Analyseergebnis optimiert werden. Vorteilhaft kann im Vergleich zu reinen Query-Systemen eine deutliche Verbesserung einer Qualität der Analyseergebnisse und eine deutliche Vergrößerung der zur Verfügung stehenden Analysemöglichkeiten erreicht werden.

Alternativ oder zusätzlich dazu wird vorgeschlagen, dass die von dem lokalen Analysemodul ausführbaren Skripte, Skripte zur Erzeugung von Modellen für maschinelles Lernen sein können (vgl. u.a. die oben erwähnten komplexen Skripte), die insbesondere zur auf maschinellem Lernen basierten Auswertung der Teile der föderierten Patientendaten, auf die das lokale Analysemodul Zugriff hat, vorgesehen sind. Dadurch kann vorteilhaft ein Analyseergebnis optimiert werden. Vorteilhaft kann im Vergleich zu reinen Query-Systemen oder zu den Analyse-Skripten eine deutliche Verbesserung einer Qualität der Analyseergebnisse und eine deutliche Vergrößerung der zur Verfügung stehenden Analysemöglichkeiten erreicht werden. Es ist denkbar, dass das lokale Analysemodul dazu vorgesehen ist, Analyse-Skripte und Skripte zur Erzeugung von Modellen für maschinelles Lernen auszuführen, je nachdem was für Skripte dem lokalen Analysemodul bereitgestellt werden. Wenn das lokale Analysemodul in eine einfache Hardwareumgebung eingebettet ist, dann könnten hauptsächlich oder ausschließlich Analyse-Skripte (die genannten einfachen und umfangreicheren Skripte) ausführbar sein. Wenn das lokale Analysemodul in eine leistungsfähigere Hardwareumgebung eingebettet ist, beispielsweise in eine Hardwareumgebung mit einem GPU, dann können zusätzlich auch Skripte zur Erzeugung von Modellen für maschinelles Lernen (die genannten komplexen Skripte) ausführbar sein. Insbesondere können diese komplexen Skripte durch eine Ausführung in der Analyseumgebung ein in der jeweiligen Analyseumgebung operierendes Machine-Learning-Modell erzeugen, welches Zugriff auf die jeweiligen lokalen Patientendatenbanken besitzt. Insbesondere ist ein derartig erzeugtes Machine-Learning-Modell einer Analyseumgebung ebenfalls lokal beschränkt und kommuniziert insbesondere nicht mit Machine-Learning-Modellen anderer Analyseumgebungen. Insbesondere sind diese Machine-Learning-Modelle zur Wahrung einer hohen Datensicherheit nicht für ein sogenanntes Federated Learning vorgesehen.

Es wird demnach in diesem Zusammenhang vorgeschlagen, dass die Modelle für maschinelles Lernen auf die jeweilige Analyseumgebung, vorzugsweise das jeweilige lokale Analysemodul, beschränkt sind. Dadurch kann vorteilhaft eine besonders hohe Datensicherheit gewährleistet werden. Vorteilhaft kann ein besonders sicherer Machine-Learning-Ansatz zur Auswertung von sensiblen Patientendaten geschaffen werden. Insbesondere findet in diesem Fall lediglich ein On-Site-Training der Machine-Learning-Modelle statt und es wird insbesondere auf das Federated Learning, also den Austausch zwischen mehreren Machine-Learning-Modellen aus verschiedenen Analyseumgebungen verzichtet.

Überdies wird vorgeschlagen, dass die Analyseumgebungen jeweils zumindest ein Ausgabemodul umfassen, welches zu einer Ausgabe von Analyseergebnissen der lokalen Analysemodule vorgesehen ist, wobei jegliche Ausgabe von Analyseergebnissen nach extern, z.B. an das externe Anwender- und/oder Programmier-Schnittstellensystem, auf eine Ausgabe an, z.B. in einer Whitelist, vorab festgelegte externe Server beschränkt ist. Dadurch kann vorteilhaft eine Datensicherheit weiter erhöht werden. Vorteilhaft kann verhindert werden, dass Analyseergebnisse an unbekannte und/oder unautorisierte Quellen ausgegeben werden. Vorteilhaft kann eine Systemarchitektur mit einer zusätzlichen Sicherheitsstufe erhalten werden. Beispielsweise kann verhindert werden, dass falls auf irgendeinem Weg ein missbräuchliches Skript auf die Analyseumgebung gelangt ist, dieses dann Daten an eine unbekannte und/oder unautorisierte Quelle verschickt. Diese Daten könnten dann vorteilhaft auf dem externen Server abgefangen werden. Bevorzugt ist jedes der Ausgabemodule als ein software- und/oder hardwaretechnisches Modul ausgebildet, welches in dem jeweiligen Gesundheitsinformationssystem / Spitalinformationssystem installiert wurde. Das jeweilige Ausgabemodul ist vorzugsweise zumindest teilweise in die jeweilige Analyseumgebung integriert ausgebildet. Insbesondere kann das jeweilige Ausgabemodul zumindest teilweise eine Hardware eines Computersystems der jeweiligen Analyseumgebung, z.B. einen Prozessor oder einen Speicher, verwenden und/oder mit anderen Modulen der jeweiligen Analyseumgebung teilen. Alternativ kann das jeweilige Ausgabemodul auch zumindest teilweise als separater Server innerhalb des Computersystems der Analyseumgebung und/oder innerhalb des Intranets des jeweiligen Spitalinformationssystems implementiert sein. Insbesondere ist der externe Server als ein Proxyserver, insbesondere als ein Whitelisted-Proxyserver, ausgebildet. Insbesondere kann der externe Server einen Teil des externen Anwender- und/oder Programmier-Schnittstellensystems ausbilden. Alternativ ist jedoch auch eine davon getrennte Ausbildung denkbar.

Wenn außerdem jegliche Ausgabe von Analyseergebnissen nach extern, z.B. an das externe Anwender- und/oder Programmier-Schnittstellensystem, auf aggregierte Daten / Reporte / Ergebnisse beschränkt ist, kann vorteilhaft eine hohe Datensicherheit, insbesondere ein hoher Schutz sensibler Daten, wie Patientendaten, erreicht werden. Insbesondere umfassen die aggregierten Daten nichts, was einen Rückschluss auf eine reale Person / einen realen Patienten erlaubt. Vorzugsweise umfassen die aggregierten Daten lediglich Zahlen, (binäre) Ja/Nein-Aussagen und/oder Einzelbuchstaben, welche beispielsweise eine Kategorie oder dergleichen angeben. Insbesondere übermitteln die jeweiligen Ausgabemodule die aggregierten Daten / Reporte / Ergebnisse an das externe Anwender- und/oder Programmier-Schnittstellensystem, insbesondere an eine Ausgabeeinheit und/oder ein Dashboard des externen Anwender- und/oder Programmier-Schnittstellensystems.

In einem nicht zum Kern der Erfindung gehörenden, jedoch ebenfalls denkbaren alternativen Analysesystem könnten jegliche Ausgaben von Analyseergebnissen nach extern, z.B. an das externe Anwender- und/oder Programmier-Schnittstellensystem, auf aggregierte Daten und zusätzlich auf mittels einer Verschleierungstechnik (Obscuration Technique), wie z.B. einer Differential Privacy Sparse Vector Technique, einer Differentially-Private Stochastic Gradient Descent Technique oder dergleichen, unrückrechenbar gemachten Modelldaten / Modellparametern von Modellen für maschinelles Lernen beschränkt sein. In diesem Fall könnte eine Verfolgung eines Federated-Learning-Ansatzes ermöglicht werden. Die ausgesandten Modelldaten / Modellparameter könnten dann zur Ausbildung eines Feedback-Loops wieder von anderen Analyseumgebungen von dem externen Anwender- und/oder Programmier-Schnittstellensystem abgeholt werden oder in dem externen Anwender- und/oder Programmier-Schnittstellensystem zusammengeführt / gemittelt werden. Zudem könnten die Modelldaten / Modellparameter der jeweiligen Modelle verglichen werden, um Kohortenunterschiede feststellen zu können.

Zusätzlich wird vorgeschlagen, dass das Analysesystem, insbesondere jede der Analyseumgebungen des Analysesystems, lokale Datenextraktionsmodule umfassen, welche dazu vorgesehen sind, Patientenakten eines Spitalinformationssystems auszulesen und daraus den jeweils der Analyseumgebung zugeordneten Teil der föderierten Patientendaten zu erstellen, auf den die jeweiligen lokalen Analysemodule Zugriff besitzen. Dadurch kann insbesondere eine vorteilhafte Systemarchitektur bereitgestellt werden. Das Datenextraktionsmodul kann beispielsweise das Anonymisierungsmodul umfassen. Bevorzugt ist jedes der Datenextraktionsmodule als ein software- und/oder hardwaretechnisches Modul ausgebildet, welches in dem jeweiligen Gesundheitsinformationssystem / Spitalinformationssystem installiert wurde. Das jeweilige Datenextraktionsmodul ist vorzugsweise zumindest teilweise in die jeweilige Analyseumgebung integriert ausgebildet. Insbesondere kann das jeweilige Datenextraktionsmodul zumindest teilweise eine Hardware eines Computersystems der jeweiligen Analyseumgebung, z.B. einen Prozessor oder einen Speicher, verwenden und/oder mit anderen Modulen der jeweiligen Analyseumgebung teilen. Alternativ kann das jeweilige Datenextraktionsmodul auch zumindest teilweise als separater Server innerhalb des Computersystems der Analyseumgebung und/oder innerhalb des Intranets des jeweiligen Spitalinformationssystems implementiert sein. Insbesondere speichert das Datenextraktionsmodul die extrahierten Daten in der lokalen Patientendatenbank der lokalen Analyseumgebung ab. Insbesondere hat die Analyseumgebung keinen direkten Zugriff auf Patientenakten des jeweiligen Spitalinformationssystems. Das lokale Datenextraktionsmodul kann dazu vorgesehen sein, die extrahierten Daten derart aufzubereiten und in der lokalen Patientenanalysedatenbank abzuspeichern, dass diese gut von Skripten auslesbar / bearbeitbar sind, z.B. in einer Tabellenform.

Wenn das Datenextraktionsmodul, insbesondere das Anonymisierungsmodul, eine Anonymisierungs- und/oder De-Identifizierungsroutine aufweist, welche dazu vorgesehen ist, bei dem Auslesen der Patientenakten und dem Erstellen der den föderierten Patientendaten zugeordneten Daten alle in der ursprünglichen Patientenakte enthaltenen Datenmerkmale, die eine Zuordnung zu einem Individuum erlauben, zu entfernen und/oder zu verschleiern, kann vorteilhaft eine besonders hohe Datensicherheit, insbesondere durch einen mehrstufigen Schutz, erreicht werden. Vorteilhaft kann eine besonders datenschutzwahrende Systemarchitektur geschaffen werden. Insbesondere speichert das Datenextraktionsmodul lediglich entsprechend der Anonymisierungs- und/oder De-Identifizierungsroutine de-identifizierte und/oder anonymisierte Patientendaten in der lokalen Patientenanalysedatenbank ab.

Des Weiteren wird vorgeschlagen, dass die Analyseumgebungen jeweils eine lokale Benutzerschnittstelle umfassen, welche eine On-Site-Analyse des lokal vorhandenen Teils der föderierten Patientendaten erlaubt und/oder welche eine On-Site-Ausführung der Skripte auf den lokal vorhandenen Teil der föderierten Patientendaten ermöglicht. Dadurch kann vorteilhaft eine hohe Benutzerfreundlichkeit erreicht werden. Insbesondere stellt die lokale Benutzerschnittstelle ein lokales Dashboard bereit. Insbesondere ist die lokale Benutzerschnittstelle ausschließlich über das Intranet der jeweiligen Gesundheitsinstitutionen / Krankenhäuser zugreifbar. Gegebenenfalls kann eine Zugriffsmöglichkeit auf die lokale Benutzerschnittstelle über einen VPN-Tunnel in das Intranet der jeweiligen Gesundheitsinstitutionen / Krankenhäuser vorgesehen sein. Insbesondere ist die lokale Benutzerschnittstelle für externe Entwickler, insbesondere für Ersteller und Bereitsteller von Skripten mittels des externen Anwender- und/oder Programmier-Schnittstellensystems, unzugänglich.

Außerdem werden die Analyseumgebung und das Anwender- und/oder Programmier-Schnittstellensystem des Analysesystems vorgeschlagen, womit eine vorteilhafte Systemarchitektur erreicht werden kann.

Ferner wird ein datenschutzwahrendes Analyseverfahren für föderierte Patientendaten, insbesondere zu einer Rekrutierung von Probanden für klinische Studien, zu einer Ermittlung von Trends, zur einer Erlangung von Optimierungsvorschlägen für Behandlungen, zu einer Umsetzung von klinischen Dashboards und/oder zur Modellierung von Verfahren des maschinellen Lernens, mittels des Analysesystems vorgeschlagen, wobei über das externe Anwender- und/oder Programmier-Schnittstellensystem die von den lokalen Analysemodulen ausführbaren Skripte erstellt und/oder bereitgestellt werden und wobei Daten und Skripte zwischen den lokalen Analysemodulen und dem externen Schnittstellensystem ausschließlich über von der jeweiligen Analyseumgebung ausgehende Verbindungen, insbesondere Outbound-Verbindungen, transferiert werden. Dadurch kann vorteilhaft eine auf der Ausführung von Skripten basierende datenschutzwahrende Analysemöglichkeit bereitgestellt werden.

Das erfindungsgemäße Analysesystem, die erfindungsgemäße Analyseumgebung, das erfindungsgemäße Anwender und Programmier-Schnittstellensystem und das erfindungsgemäße Analyseverfahren sollen hierbei nicht auf die oben beschriebene Anwendung und Ausführungsform beschränkt sein. Insbesondere kann das erfindungsgemäße Analysesystem, die erfindungsgemäße Analyseumgebung, das erfindungsgemäße Anwender und Programmier-Schnittstellensystem und das erfindungsgemäße Analyseverfahren zu einer Erfüllung einer hierin beschriebenen Funktionsweise eine von einer hierin genannten Anzahl von einzelnen Elementen, Bauteilen, Verfahrensschritten und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer übergreifenden Systemarchitektur eines datenschutzwahrenden Analysesystems für föderierte Patientendaten,
- Fig. 2: eine schematische Darstellung des datenschutzwahrenden Analysesystems inklusive einer Illustration wichtiger Schritte eines das Analysesystem anwendenden datenschutzwahrenden Analyseverfahrens für föderierte Patientendaten,
- Fig. 3: eine beispielhafte Ausgabe aggregierter Analyseergebnisse eines Ausgabemoduls des Analysesystems,
- Fig. 4: eine beispielhafte Ausgabe einer lokalen Benutzerschnittstelle einer lokalen Analyseumgebung des Analysesystems und
- Fig. 5: ein schematisches Ablaufdiagramm des datenschutzwahrenden Analyseverfahrens mittels des Analysesystems.

### Beschreibung der Ausführungsbeispiele

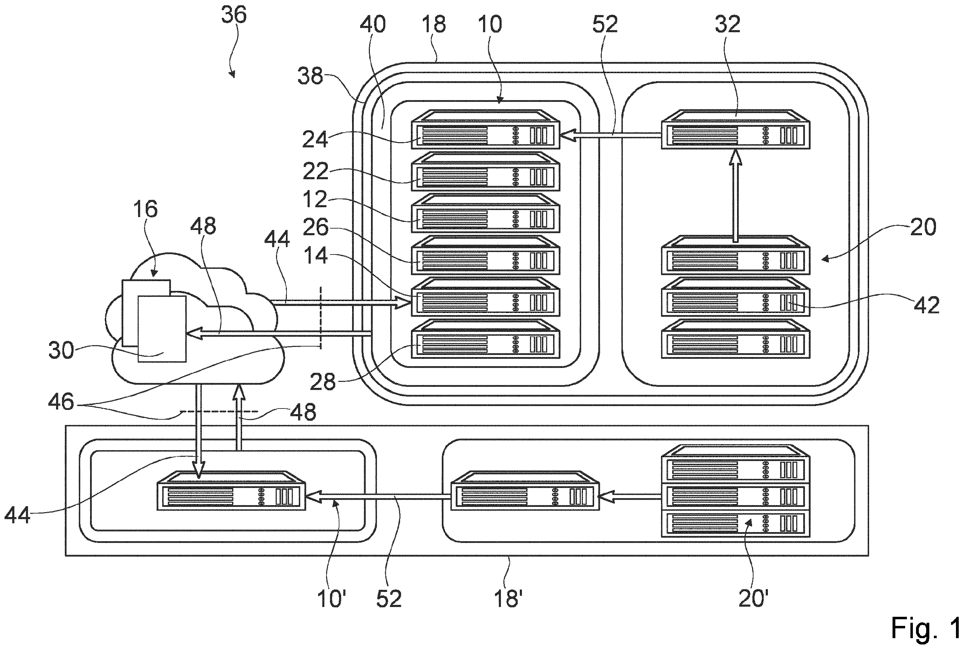

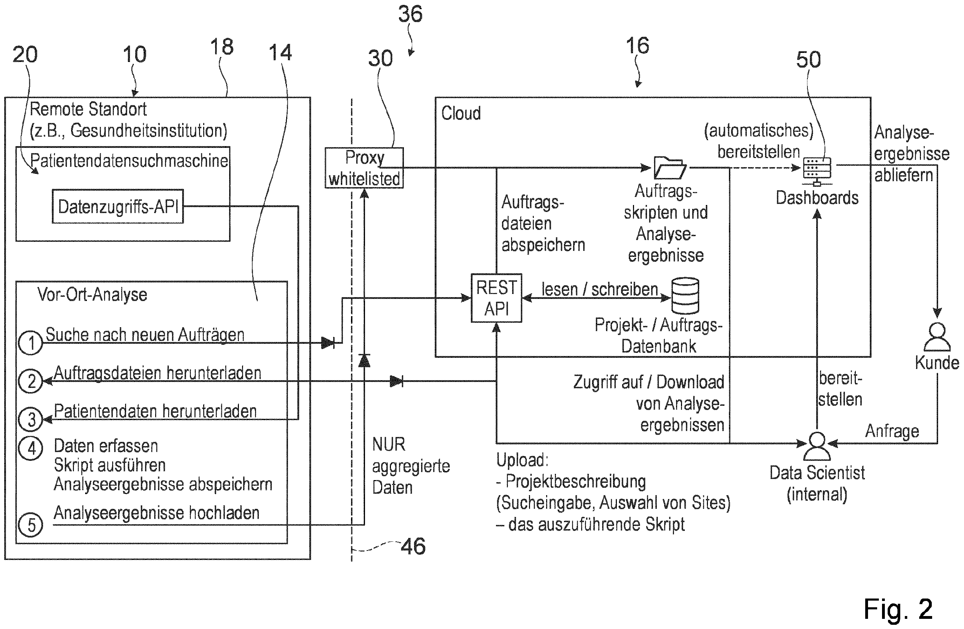

Die Figuren 1 und 2 zeigen jeweils schematische Darstellungen eines datenschutzwahrenden Analysesystems 36 für föderierte Patientendaten. Das Analysesystem 36 weist eine Mehrzahl an Analyseumgebungen 10, 10' auf. Beispielhaft sind in der Fig. 1 zwei Analyseumgebungen 10, 10' dargestellt, es können jedoch auch deutlich mehr als zwei Analyseumgebungen 10, 10' an dem Analysesystem 36 beteiligt sein. Die Analyseumgebungen 10, 10' sind voneinander räumlich getrennt angeordnet. Die Analyseumgebungen 10, 10' sind systemtechnisch voneinander getrennt. Die Analyseumgebungen 10, 10' des Analysesystems 36 sind jeweils in innerhalb von speziell gesicherten und/oder zugangsbeschränkten Bereichen 18, 18' von Gesundheitsinformationssystemen 20, 20' angeordnet. Die speziell gesicherten und/oder zugangsbeschränkten Bereiche 18, 18' werden jeweils durch Intranets der jeweiligen Gesundheitsinformationssysteme 20, 20' ausgebildet. Die Gesundheitsinformationssysteme 20, 20' können jeweils als Spitalinformationssysteme (HIS) ausgebildet sein oder als andere Informationssysteme anderer Gesundheitsinstitutionen. Der speziell gesicherte und/oder zugangsbeschränkte Bereich 18 ist durch eine - Gesundheitsinformationssystems-Firewall 38 geschützt. Zudem weist die Analyseumgebung 10 einer weitere Analyseumgebungs-Firewall 40 auf, welche zu einem Schutz der Analyseumgebung 10 vorgesehen ist. Die Module und Komponenten der Analyseumgebung 10 befinden sich somit hinter wenigstens zwei Firewalls 38, 40, der Gesundheitsinformationssystems-Firewall 38 und der Analyseumgebungs-Firewall 40. Das Gesundheitsinformationssystem 20, 20' ist frei von für die Analyseumgebungen 10, 10' offenen Ports, insbesondere frei von für die Analyseumgebungen 10, 10' offenen VPN-Ports oder Inbound-Verbindungsports. In die Figur 2 ist eine Trennlinie 46 eingezeichnet, welche eine Grenze zwischen speziell gesichertem und/oder zugangsbeschränktem Bereich 18, 18' bzw. Intranet (hier: links der Trennlinie 46) und öffentlich zugänglicher Bereich bzw. Internet anzeigen soll.

Das Gesundheitsinformationssystem 20, 20' umfasst eine Patientenakten-Datenbank 42. Die Patientendaten-Datenbank 42 ist außerhalb der Analyseumgebung 10, 10' angeordnet. Die Analyseumgebung 10, 10' besitzt keine Zugriffsberechtigung für einen Zugriff auf die Patientendaten-Datenbank 42 des jeweiligen Gesundheitsinformationssystems 20, 20'. In der Patientendaten-Datenbank 42 sind die originalen Patientenakten der Gesundheitsinstitution abgespeichert. Diese Patientenakten umfassen hochsensible Daten. Die Analyseumgebungen 10, 10' umfassen jeweils eine lokale Patientenanalysedatenbank 12. Die lokalen Patientenanalysedatenbanken 12 sind dazu vorgesehen, jeweils einen Teil der durch das Analysesystem 36 auswertbaren föderierten Patientendaten zu umfassen, insbesondere abzuspeichern. Die auf den Patientenanalysedatenbanken 12 abgespeicherten föderierten Patientendaten entsprechen nicht den Patientenakten des jeweiligen Gesundheitsinformationssystems 20, 20'. Die auf den Patientenanalysedatenbanken 12 abgespeicherten föderierten Patientendaten sind lediglich basierend auf den Patientenakten des jeweiligen Gesundheitsinformationssystems 20, 20' erstellt.

Die Analyseumgebungen 10, 10' umfassen jeweils ein lokales Analysemodul 14. Die lokalen Analysemodule 14 sind zumindest zu einer Ausführung von Skripten auf den in der jeweiligen lokalen Patientenanalysedatenbank 12 verfügbaren Teil der föderierten Patientendaten vorgesehen. Die lokalen Analysemodule 14 können zudem zu einem einfachen Durchsuchen der in den jeweiligen lokalen Patientenanalysedatenbanken 12 verfügbaren Teile der föderierten Patientendaten basierend auf einfachen Suchstrings vorgesehen sein. Die von dem lokalen Analysemodul 14 ausführbaren Skripte können Analyse-Skripte zur rein algorithmischen (Machine-Learning unabhängigen) Auswertung der Teile der föderierten Patientendaten, auf die das lokale Analysemodul 14 Zugriff hat, sein. Zudem können die von dem lokalen Analysemodul 14 ausführbaren Skripte, Skripte zur Erzeugung von Modellen für maschinelles Lernen sein. Die Skripte zur Erzeugung von Modellen für maschinelles Lernen sind zu einer lokalen Erzeugung von Maschinelles-Lernen-Modulen in den Analyseumgebungen 10, 10' vorgesehen. Die Skripte zur Erzeugung von Modellen für maschinelles Lernen sind zu einer auf maschinellem Lernen basierten Auswertung der Teile der föderierten Patientendaten, auf die das lokale Analysemodul 14 Zugriff hat, vorgesehen. Die durch die Skripte lokal erzeugten Modelle für maschinelles Lernen sind auf das jeweilige lokale Analysemodul 14 beschränkt.

Das Analysesystem 36 umfasst in jedem der Gesundheitsinformationssysteme 20, 20' ein lokales Datenextraktionsmodul 32. Es ist denkbar, dass das lokale Datenextraktionsmodul 32 jeweils der Analyseumgebung 10, 10' des jeweiligen Gesundheitsinformationssystems 20, 20' zugeordnet ist. Alternativ kann das lokale Datenextraktionsmodul 32 jedoch auch, wie in der Fig. 1 beispielhaft dargestellt, ein außerhalb der Analyseumgebung 10, 10' angeordnetes Schnittstellenmodul zwischen der Patientendaten-Datenbank 42 des Gesundheitsinformationssystems 20, 20' und der Analyseumgebung 10, 10' des Gesundheitsinformationssystems 20, 20' sein. Das lokale Datenextraktionsmodul 32 ist dazu vorgesehen, die Patientenakten des jeweils zugehörigen Gesundheitsinformationssystems 20, 20' auszulesen und daraus den jeweils der Analyseumgebung 10, 10' zugeordneten Teil der föderierten Patientendaten zu erstellen, auf den die jeweiligen lokalen Analysemodule 14 Zugriff besitzen und welcher in der lokalen Patientenanalysedatenbank 12 abgespeichert ist. Das lokale Datenextraktionsmodul 32 kann als ein lokaler ETL (Extract-Transfer-Load)-Server ausgebildet sein. Das Datenextraktionsmodul 32 kann als ein verschlüsselter Server ausgebildet sein. Das lokale Datenextraktionsmodul 32 weist eine Anonymisierungs- und/oder De-Identifizierungsroutine auf. Die Anonymisierungs- und/oder De-Identifizierungsroutine des Datenextraktionsmoduls 32 ist dazu vorgesehen, bei dem Auslesen der Patientenakten aus der Patientenakten-Datenbank 42 und dem Erstellen der den föderierten Patientendaten zugeordneten Daten / der in der Patientenanalysedatenbank 12 der Analyseumgebung 10, 10' abgespeicherten Daten alle in der ursprünglichen Patientenakte enthaltenen Datenmerkmale, die eine Zuordnung zu einem Individuum erlauben, zu entfernen (bspw. durch De-identifikation) und/oder zu verschleiern (bspw. durch Anonymisierung). Das lokale Datenextraktionsmodul 32 ist dazu vorgesehen, die de-identifizierten und/oder anonymisierten Patientendaten an die Patientenanalysedatenbank 12 zu übertragen und dort abzuspeichern (vgl. Pfeil 52).

Das Analysesystem 36 weist ein Schnittstellensystem 16 auf. Das Schnittstellensystem 16 bildet ein Anwender- und/oder Programmier-Schnittstellensystem 16 aus. Das Schnittstellensystem 16 ist ein externes Schnittstellensystem 16, welches räumlich und systemtechnisch von jeder Analyseumgebung 10, 10' des Analysesystems 36 getrennt ausgebildet und angeordnet ist. Das externe Anwender- und/oder Programmier-Schnittstellensystem 16 ist außerhalb der speziell gesicherten und/oder zugangsbeschränkten Bereiche 18 der Gesundheitsinformationssysteme 20, 20' angeordnet. Das externe Anwender- und/oder Programmier-Schnittstellensystem 16 ist außerhalb der Intranets der Gesundheitsinformationssysteme 20, 20' angeordnet. Das externe Anwender- und/oder Programmier-Schnittstellensystem 16 kann zentral angeordnet und ausgebildet sein. In dem in der Fig. 1 dargestelltem Ausführungsbeispiel ist das Anwender- und/oder Programmier-Schnittstellensystem 16 als verteilt angeordnetes, cloudbasiertes System ausgebildet.

Das externe Anwender- und/oder Programmier-Schnittstellensystem 16 ist dazu vorgesehen, die von den lokalen Analysemodulen 14 ausführbaren Skripte von extern für die Analyseumgebungen 10, 10' bereitzustellen. Das externe Anwender- und/oder Programmier-Schnittstellensystem 16 stellt die von den lokalen Analysemodulen 14 ausführbaren Skripte an bekannten und vertrauenswürdigen Stellen / Adressen für einen Download durch die Analyseumgebungen 10, 10' bereit. Das externe Anwender- und/oder Programmier-Schnittstellensystem 16 ermöglicht eine externe Erstellung der von den lokalen Analysemodulen 14 ausführbaren Skripte. Das externe Anwender- und/oder Programmier-Schnittstellensystem 16 weist ein Anwender- und/oder Programmierer-Interface auf, welches eine Erstellung und/oder Auswahl der bereitzustellenden Skripte ermöglicht. Das externe Anwender- und/oder Programmier-Schnittstellensystem 16 weist eine Skript-Testumgebung auf. Die Skript-Testumgebung ist dazu vorgesehen, neue Skripte oder sich in der Entwicklung befindliche Skripte vor einer Bereitstellung zur Übergabe an die Analyseumgebung/en 10, 10' zu testen. Das externe Anwender- und/oder Programmier-Schnittstellensystem 16 weist dazu künstliche Testumgebungs-Patientendaten auf, welche für den Test der Skripte vorgesehen sind.

Das externe Anwender- und/oder Programmier-Schnittstellensystem 16 ist mit jeder der Analyseumgebungen 10, 10' datentransfertechnisch ausschließlich über von den Analyseumgebungen 10, 10' ausgehende Verbindungen verbunden. Der Datentransfer zwischen dem externen Anwender- und/oder Programmier-Schnittstellensystem 16 und den Analyseumgebungen 10, 10' erfolgt ausschließlich über von den Analyseumgebungen 10, 10' ausgehende Verbindungen. Die einzigen möglichen Verbindungen zwischen dem externen Anwender- und/oder Programmier-Schnittstellensystem 16 und den Analyseumgebungen 10, 10' sind von den Analyseumgebungen 10, 10' ausgehende Outbound-Verbindungen.

Die Analyseumgebungen 10, 10' weisen jeweils zumindest ein Skript-Abholmodul 22 auf. Das Skript-Abholmodul 22 ist dazu vorgesehen, eine, insbesondere regelmäßige, Abfrage des externen Schnittstellensystems 16 nach neuen, von dem externen Anwender- und/oder Programmier-Schnittstellensystem 16 für die lokalen Analysemodule 14 bereitgestellten, Skripten durchzuführen. Das Skript-Abholmodul 22 ist dazu vorgesehen, bei einem Auffinden von neu bereitgestellten Skripten mittels der von den Analyseumgebungen 10, 10' ausgehenden Verbindungen, insbesondere der Outbound-Verbindungen, einen Download des/der bereitgestellten Skripts/Skripte von dem externen Schnittstellensystem 16 zu veranlassen.

Die Analyseumgebungen 10, 10' weisen jeweils eine lokale Programmbibliothek-Datenbank 24 auf. Die lokalen Programmbibliothek-Datenbanken 24 beinhalten Programmbibliotheken und/oder Software-Hilfsmodule, die von Skripten aufrufbar sind. Die Analyseumgebungen 10, 10' weisen softwaretechnische und/oder hardwaretechnische Vorkehrungen auf, die ein durch die Skripte initiiertes Aufrufen und/oder Herunterladen von externen Programmbibliotheken und/oder Software-Hilfsmodulen, die nicht in der lokalen Programmbibliothek-Datenbank 24 umfasst sind, unterbinden. Die Analyseumgebungen 10, 10' weisen softwaretechnische und/oder hardwaretechnische Vorkehrungen auf, die ein Aufrufen und/oder Herunterladen von externen Programmbibliotheken und/oder Software-Hilfsmodulen aus dem Internet, insbesondere von unbekannten, nicht dem externen Anwender- und/oder Programmier-Schnittstellensystem 16 zugehörigen/zugeordneten Stellen und/oder Adressen, unterbinden. Die Analyseumgebungen 10, 10' umfassen jeweils zumindest ein Bibliothek-Abholmodul 26. Das Bibliothek-Abholmodul 26 ist dazu vorgesehen, eine, insbesondere regelmäßige, Abfrage des externen Schnittstellensystems 16 nach neuen, von dem externen Schnittstellensystem 16 für die lokale Programmbibliothek-Datenbank 24 bereitgestellten, insbesondere autorisierten, externen Programmbibliotheken und/oder Software-Hilfsmodulen durchzuführen. Das Bibliothek-Abholmodul 26 ist dazu vorgesehen, bei einem Auffinden einer/eines neu bereitgestellten insbesondere autorisierten, externen Programmbibliothek und/oder Software-Hilfsmoduls mittels der von den Analyseumgebungen 10, 10' ausgehenden Verbindungen, vorzugsweise der Outbound-Verbindungen, einen Download der/des bereitgestellten Programmbibliothek und/oder Software-Hilfsmoduls, vorzugsweise von dem externen Schnittstellensystem 16 und/oder von einer vertrauenswürdigen und/oder bekannten Stelle oder Adresse, zu veranlassen. In der Figur 1 ist der Download von Skripten, Programmbibliotheken und/oder Software-Hilfsmodulen von dem Anwender- und/oder Programmier-Schnittstellensystem 16 zu den Analyseumgebungen 10, 10' durch einen Pfeil 44 dargestellt. Dieser durch den Pfeil 44 dargestellte Kommunikationspfad ist auf die Outbound-Verbindungen beschränkt. Dies wird in der Figur 2 durch die Verwendung von Dioden-Schaltsymbolen verdeutlicht. Kommunikationspfade, die in der Figur 2 mit Dioden-Schaltsymbolen versehen sind, sind Einweg-Kommunikationspfade, bei denen die Zielseite (die Seite, in welche die Spitze des Dreiecks des Dioden-Schaltsymbols zeigt, also die rechts der Trennlinie 46 liegende Seite der Figur 2) nicht in der Lage ist eine Kommunikation zu initiieren. Die Initiierung einer Kommunikation geht in diesem Fall immer von der Startseite (die der Spitze des Dreiecks des Dioden-Schaltsymbols entgegenliegende Seite, also die links der Trennlinie 46 liegende Seite der Figur 2) aus.

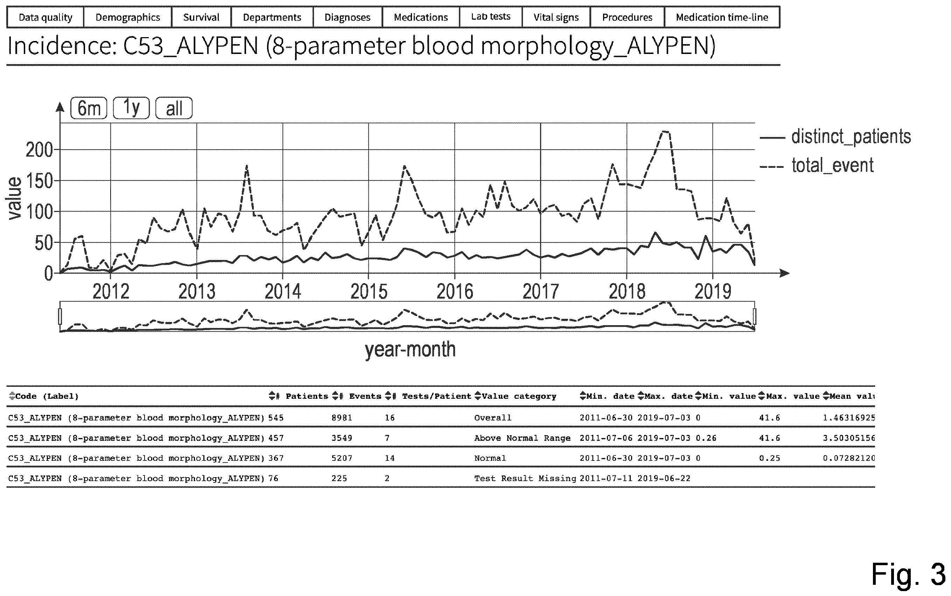

Die Analyseumgebungen 10, 10' weisen jeweils ein Ausgabemodul 28 auf. Die Ausgabemodule 28 sind zu einer Ausgabe von Analyseergebnissen der lokalen Analysemodule 14 vorgesehen. Die Ausgabemodule 28 sind zu einer Ausgabe der mit Hilfe der Skripte von den lokalen Analysemodulen 14 erzeugten Analyseergebnisse vorgesehen. Das Analysesystem 36 umfasst wenigstens einen externen Server 30. Der externe Server 30 ist als ein Proxyserver ausgebildet. Die Ausgabemodule 28 sind dazu vorgesehen, jegliche Ausgabe von Analyseergebnissen nach extern, z.B. an das externe Anwender- und/oder Programmier-Schnittstellensystem 16, auf eine Ausgabe nur an die vorab festgelegten externen Server 30 zu beschränken. Die Ausgabemodule 28 umfassen zu diesem Zweck eine Whitelist. Die Whitelist umfasst alle externen Server 30, an welche ein Versenden von Analyseergebnissen der Analysemodule 14 möglich ist. Jegliche Ausgabe von Analyseergebnissen nach extern, z.B. an das externe Anwender- und/oder Programmier-Schnittstellensystem 16, ist vollständig auf aggregierte Daten beschränkt. Die Ausgabemodule 28 sind dazu vorgesehen, jegliche Ausgabe von Analyseergebnissen nach extern, z.B. an das externe Anwender- und/oder Programmier-Schnittstellensystem 16, auf ausschließlich aggregierte Daten zu beschränken. In der Figur 1 ist die Ausgabe der Analyseergebnisse durch einen Pfeil 48 dargestellt. Über den Pfad des Pfeils 48 verlassen lediglich aggregierte Daten, die keine Rückschlüsse auf echte Patienten zulassen, die Analyseumgebungen 10, 10' bzw. die Gesundheitsinformationssysteme 20, 20'. In der Figur 3 ist eine beispielhafte Ausgabe aggregierter Analyseergebnisse des Ausgabemoduls 28 dargestellt. Diese Ausgabe ist von extern, z.B. über das Internet, beispielsweise mittels eines zentralen Dashboards 50 des Anwender- und/oder Programmier-Schnittstellensystems 16 (vgl. Fig. 2) abrufbar.

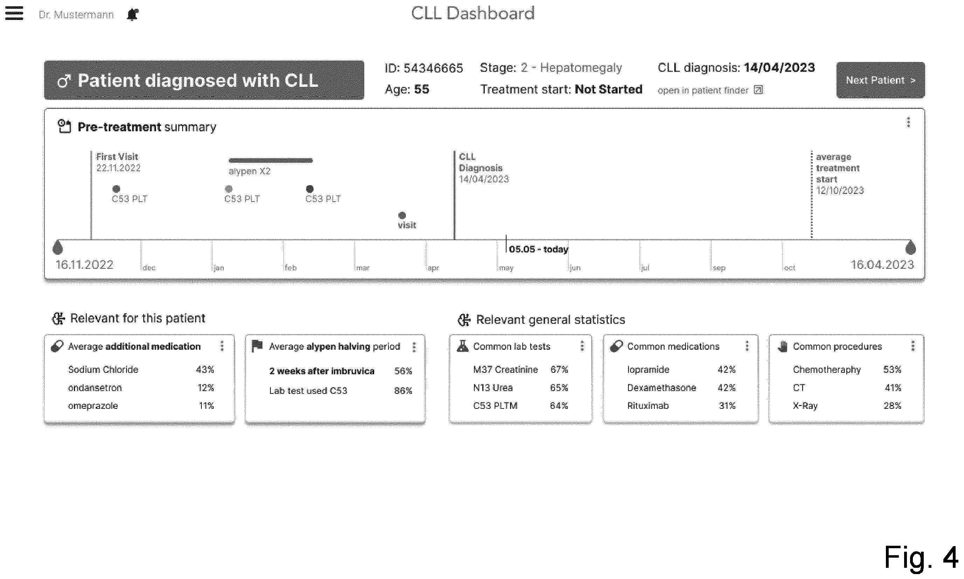

Die Analyseumgebungen 10, 10' umfassen jeweils eine lokale Benutzerschnittstelle 34 (vgl. Fig. 2). Die lokale Benutzerschnittstelle 34 ist als ein lokales Dashboard ausgebildet. Die lokale Benutzerschnittstelle 34 erlaubt eine On-Site-Analyse des lokal in der jeweiligen Patientenanalysedatenbank 12 vorhandenen Teils der föderierten Patientendaten. Die lokale Benutzerschnittstelle 34 ermöglicht eine On-Site-Ausführung der Skripte auf den in der Patientenanalysedatenbank 12 lokal vorhandenen Teil der föderierten Patientendaten. In der Figur 4 ist eine beispielhafte Ausgabe der lokalen Benutzerschnittstelle 34 dargestellt, welche einen Dateneintrag aus der Patientenanalysedatenbank 12, der einem de-identifizierten Patienten zugehörig ist, wiedergibt. Diese Anzeige ist lediglich von innerhalb des gesicherten Bereichs 18, 18' der jeweiligen Gesundheitsinstitution möglich.

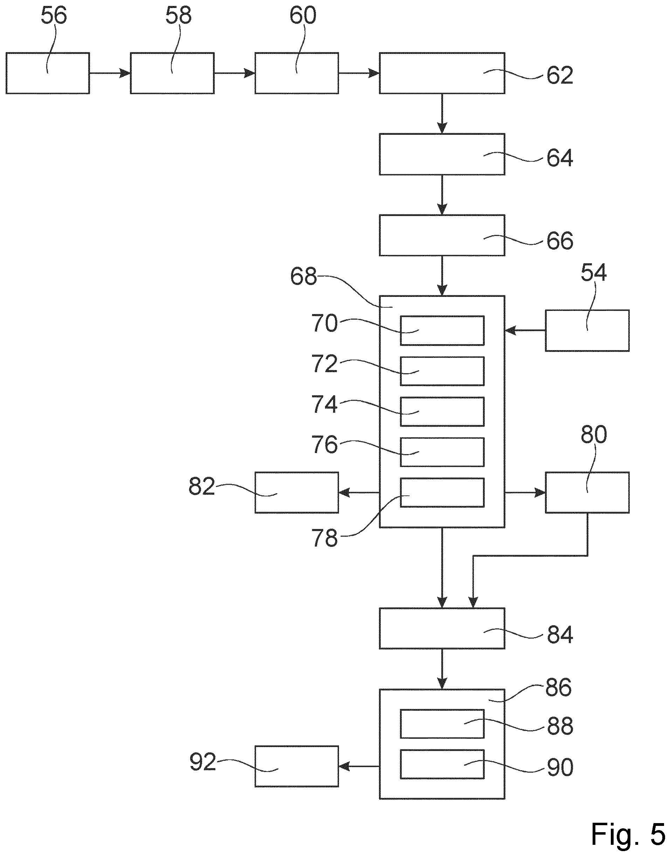

Die Figur 5 zeigt ein schematisches Ablaufdiagramm eines datenschutzwahrenden Analyseverfahrens für föderierte Patientendaten mittels des Analysesystems 36. In zumindest einem Verfahrensschritt 54 werden mittels den jeweiligen lokalen Datenextraktionsmodulen 32 Patientendaten aus den Patientenakten der Gesundheitsinformationssysteme 20, 20' kopiert, modifiziert und in modifizierter Form in den lokalen Patientenanalysedatenbanken 12 abgespeichert. In dem Verfahrensschritt 54 werden dabei die Patientendaten mittels der Anonymisierungs- und/oder De-Identifizierungsroutine derart modifiziert, dass alle in der ursprünglichen Patientenakte enthaltenen Datenmerkmale, die eine Zuordnung zu einem Individuum erlauben, entfernt und/oder verschleiert werden. In den einzelnen lokalen Patientenanalysedatenbanken 12 sind somit jeweils nur anonymisierte und/oder de-identifizierte Patientendaten aus dem zu der jeweiligen lokalen Patientenanalysedatenbank 12 zugehörigen Gesundheitsinformationssystem 20, 20' abgespeichert. Die auf den lokalen Patientenanalysedatenbanken 12 vorhandenen anonymisierten und/oder de-identifizierten Patientendaten stehen dann für die Analysen mittels der Analysemodule 14 zur Verfügung. In einem Verfahrensschritt 56 wird außerhalb der Analyseumgebungen 10, 10' und außerhalb der Gesundheitsinformationssysteme 20, 20' ein Skript erstellt. Das Skript kann u.a. ein Analyse-Skript oder ein Skript zur Erzeugung von Modellen für maschinelles Lernen sein. Das Skript kann mittels des externe Anwender- und/oder Programmier-Schnittstellensystems 16 erstellt werden oder an das externe Anwender- und/oder Programmier-Schnittstellensystem 16 übergeben werden. In zumindest einem Verfahrensschritt 58 wird das erstellte oder empfangene Skript in der Skript-Testumgebung des externen Anwender- und/oder Programmier-Schnittstellensystems 16 anhand der künstlichen Testumgebungs-Patientendaten getestet. In zumindest einem weiteren Verfahrensschritt 60 wird das Skript einer manuellen oder automatisierten Sicherheitsüberprüfung unterworfen.

In zumindest einem weiteren Verfahrensschritt 62 wird das, vorzugsweise sicherheitsgeprüfte, Skript an einer dafür vorgesehenen Stelle/Adresse für einen Download durch die Analyseumgebungen 10, 10' bereitgehalten. Dabei kann eine Auswahl aus der Gesamtmenge der Analyseumgebungen 10, 10' getroffen werden, was einer Auswahl an Gesundheitsinstitutionen, die in der Analyse umfasst sein sollen, entspricht. In zumindest einem Verfahrensschritt 64 fragen die lokalen Skript-Abholmodule 22 die dafür vorgesehenen Stellen/Adressen des externen Anwender- und/oder Programmier-Schnittstellensystems 16 ab, um herauszufinden, ob für die ihr zugehörige Analyseumgebung 10, 10' ein oder mehrere Skripte bereitgestellt sind. Dies kann beispielsweise über eine Einweg-Kommunikation der jeweiligen Analyseumgebung 10, 10' mit einem REST API des externen Anwender- und/oder Programmier-Schnittstellensystems 16 geschehen. In zumindest einem weiteren Verfahrensschritt 66 werden die bereitgestellten Skripte durch die Skript-Abholmodule 22 der jeweiligen Analyseumgebungen 10, 10' von dem externen Anwender- und/oder Programmier-Schnittstellensystem 16 heruntergeladen. In zumindest einem weiteren Verfahrensschritt 68 werden die heruntergeladenen Skripte von den jeweiligen Analyseumgebungen 10, 10' ausgeführt. Durch die Ausführung der Skripte werden Analysen der in den lokalen Patientenanalysedatenbanken 12 abgespeicherten Patientendaten durchgeführt. Durch die Ausführung der Skripte werden die Analyseergebnisse gewonnen. Es ist denkbar, dass sich die Skripte Programmbibliotheken oder Software-Hilfsmodulen bedienen, die nicht Teil des Skripts sind, jedoch von dem Skript aufgerufen werden sollen.

In einem Teilverfahrensschritt 70 des Verfahrensschritts 68 wird bei der Ausführung des Skripts zumindest eine Programmbibliothek und/oder zumindest ein Software-Hilfsmodul aus der lokalen Programmbibliothek-Datenbank 24 der jeweiligen Analyseumgebung 10, 10' verwendet. Dies kann nur dann erfolgen, wenn die jeweils benötigte Programmbibliothek und/oder das jeweils benötigte Software-Hilfsmodul in der lokalen Programmbibliothek-Datenbank 24 vorhanden ist. Falls ein Skript in dem Verfahrensschritt 68 versucht auf eine Programmbibliothek und/oder auf ein Software-Hilfsmodul zuzugreifen, das nur extern verfügbar ist, blockiert die Analyseumgebung 10, 10' diesen Versuch und/oder sucht an einer dafür vorgesehenen, vertrauenswürdigen Stelle / Adresse des externen Anwender- und/oder Programmier-Schnittstellensystems 16 nach der gewünschten Programmbibliothek und/oder dem gewünschten Software-Hilfsmodul. Verweise von Skripten auf andere Stellen oder Adressen, an denen Programmbibliotheken und/oder Software-Hilfsmodule gefunden werden können, werden von den Analyseumgebungen 10, 10' vollständig blockiert. In einem weiteren Teilverfahrensschritt 72 des Verfahrensschritts 68 werden neue Programmbibliotheken und/oder Software-Hilfsmodule vor einer Freigabe für die Analyseumgebungen 10, 10' sicherheitsgeprüft. In einem weiteren Teilverfahrensschritt 74 des Verfahrensschritts 68 werden die sicherheitsgeprüften neuen Programmbibliotheken und/oder Software-Hilfsmodule an einer dafür vorgesehenen Stelle / Adresse des externen Anwender- und/oder Programmier-Schnittstellensystems 16 bereitgestellt. In einem weiteren Teilverfahrensschritt 76 des Verfahrensschritts 68 fragen die lokalen Bibliothek-Abholmodule 26 die dafür vorgesehenen Stellen/Adressen des externen Anwender- und/oder Programmier-Schnittstellensystems 16 ab, um herauszufinden, ob dort ein oder mehrere autorisierte Programmbibliotheken und/oder Software-Hilfsmodule bereitgestellt sind. In zumindest einem weiteren Teilverfahrensschritt 78 des Verfahrensschritts 68 werden die bereitgestellten Programmbibliotheken und/oder Software-Hilfsmodule durch die Bibliothek-Abholmodule 26 der jeweiligen Analyseumgebungen 10, 10' von dem externen Anwender- und/oder Programmier-Schnittstellensystem 16 heruntergeladen. Sobald die heruntergeladenen Programmbibliotheken und/oder Software-Hilfsmodule auf der jeweiligen lokalen Programmbibliothek-Datenbank 24 abgespeichert sind, stehen sie für die Verwendung durch Skripte lokal zur Verfügung. In zumindest einem Verfahrensschritt 80 kann ein Skript, wenn es als Skript zur Erzeugung eines Modells für maschinelles Lernen ausgebildet ist, ein lokal auf die Analyseumgebung 10, 10' beschränktes Machine-Learning-Modell erzeugen und aktivieren.

In zumindest einem Verfahrensschritt 82 wird von einem lokal am Ort der jeweiligen Gesundheitsinstitution verorteten Nutzer mittels der lokalen Benutzerschnittstelle 34 ein lokales Dashboard (vgl. Fig. 4) aufgerufen, welches eine On-Site-Durchsicht der Analyseergebnisse in nicht-aggregierter Form erlaubt. Dazu benötigt der Nutzer eine spezielle Freigabe (z.B. ein Passwort), um auf die geschützte Analyseumgebung 10, 10' zugreifen zu können.

In zumindest einem Verfahrensschritt 84 wird in den jeweiligen Analyseumgebungen 10, 10' mit Hilfe der Analysemodule 14 ein aggregiertes Analyseergebnis der durch das Skript initiierten Analyse der in der Patientenanalysedatenbank 12 abgespeicherten Patientendaten erzeugt. In zumindest einem Verfahrensschritt 86 wird das aggregierte Analyseergebnis von dem jeweiligen lokalen Ausgabemodul 28 ausgegeben. In einem Teilverfahrensschritt 88 des Verfahrensschritts 86 wird dazu zunächst überprüft, ob die Stelle / Adresse, an die das Ausgabemodul 28 / das Skript die Analyseergebnisse senden möchte, in einer Whitelist der Analyseumgebung 10, 10' aufgeführt ist. In der Whitelist stehen vertrauenswürdige Stellen / Adressen, welche Teil des externen Anwender- und/oder Programmier-Schnittstellensystems 16 sein können oder welche als von dem externen Anwender- und/oder Programmier-Schnittstellensystem 16 getrennt ausgebildete externe Server 30, z.B. Proxyserver, ausgebildet sein können. In einem weiteren Teilverfahrensschritt 90 des Verfahrensschritts 86 wird je nachdem, ob die beabsichtigte Empfangsstelle / Empfangsadresse für die Analyseergebnisse in der Whitelist vorhanden ist oder nicht, die Ausgabe der Analyseergebnisse durch das Ausgabemodul 28 gestattet oder blockiert. In zumindest einem weiteren Verfahrensschritt 92 werden die aggregierten Analyseergebnisse über das zentrale Dashboard 50 externen Nutzern des Anwender- und/oder Programmier-Schnittstellensystems 16, z.B. Endkunden oder Wissenschaftlern, zur Verfügung gestellt. Das zentrale Dashboard 50 hat dazu Zugriff auf den externen Server 30, an den die aggregierten Analyseergebnisse übermittelt wurden. In dem datenschutzwahrenden Analyseverfahren werden Daten und Skripte zwischen den lokalen Analysemodulen 14 und dem externen Anwender- und/oder Programmier-Schnittstellensystem 16 ausschließlich über von der jeweiligen Analyseumgebung 10, 10' ausgehende Verbindungen, insbesondere Outbound-Verbindungen, transferiert.

### Bezugszeichen

- 10: Analyseumgebung
- 12: Lokale Patientenanalysedatenbank
- 14: Lokales Analysemodul
- 16: Externes Schnittstellensystem
- 18: Bereich
- 20: Gesundheitsinformationssystem
- 22: Skript-Abholmodul
- 24: Programmbibliothek-Datenbank
- 26: Bibliothek-Abholmodul
- 28: Ausgabemodul
- 30: Externer Server
- 32: Lokales Datenextraktionsmodul
- 34: Lokale Benutzerschnittstelle
- 36: Analysesystem
- 38: Gesundheitsinformationssystems-Firewall
- 40: Analyseumgebungs-Firewall
- 42: Patientenakten-Datenbank
- 44: Pfeil
- 46: Trennlinie
- 48: Pfeil
- 50: Zentrales Dashboard
- 52: Pfeil
- 54: Verfahrensschritt
- 56: Verfahrensschritt
- 58: Verfahrensschritt
- 60: Verfahrensschritt
- 62: Verfahrensschritt
- 64: Verfahrensschritt
- 66: Verfahrensschritt
- 68: Verfahrensschritt
- 70: Teilverfahrensschritt
- 72: Teilverfahrensschritt
- 74: Teilverfahrensschritt
- 76: Teilverfahrensschritt
- 78: Teilverfahrensschritt
- 80: Verfahrensschritt
- 82: Verfahrensschritt
- 84: Verfahrensschritt
- 86: Verfahrensschritt
- 88: Teilverfahrensschritt
- 90: Teilverfahrensschritt
- 92: Verfahrensschritt

## Patentansprüche

1. Datenschutzwahrendes Analysesystem (36) für föderierte Patientendaten, mit zumindest einer Mehrzahl an voneinander räumlich und systemtechnisch getrennten Analyseumgebungen (10, 10'), welche jeweils zumindest eine lokale Patientenanalysedatenbank (12) mit einem Teil der föderierten Patientendaten umfassen, und welche jeweils zumindest ein lokales Analysemodul (14) umfassen, das zumindest zu einer Ausführung von Skripten auf den in der jeweiligen lokalen Patientenanalysedatenbank (12) verfügbaren Teil der föderierten Patientendaten vorgesehen ist,
und mit zumindest einem, insbesondere zentralen oder verteilt angeordneten, externen Anwender- und/oder Programmier-Schnittstellensystem (16), welches räumlich und systemtechnisch von den Analyseumgebungen (10, 10') getrennt ist, über welches die von den lokalen Analysemodulen (14) ausführbaren Skripte, insbesondere extern, erstellbar und/oder bereitstellbar sind und welches mit jeder der Analyseumgebungen (10, 10') datentransfertechnisch ausschließlich über von den Analyseumgebungen (10, 10') ausgehende Verbindungen, insbesondere Outbound-Verbindungen, verbunden ist.

2. Analysesystem (36) nach Anspruch 1, **dadurch gekennzeichnet, dass** zumindest ein Großteil der Analyseumgebungen (10, 10'), vorzugsweise alle Analyseumgebungen (10, 10'), innerhalb von speziell gesicherten und/oder zugangsbeschränkten Bereichen (18, 18'), z.B. Intranets, von Gesundheitsinformationssystemen (20, 20'), insbesondere Spitalinformationssystemen, angeordnet ist/sind **und dass** das externe Anwender- und/oder Programmier-Schnittstellensystem (16) außerhalb dieser speziell gesicherten und/oder zugangsbeschränkten Bereiche (18, 18') von Gesundheitsinformationssystemen (20, 20'), insbesondere Spitalinformationssystemen, angeordnet ist.

3. Analysesystem (36) nach einem der vorhergehenden Ansprüche, **dadurch gekennzeichnet, dass** die Analyseumgebungen (10, 10') jeweils zumindest ein Skript-Abholmodul (22) umfassen, welches dazu vorgesehen ist, eine, insbesondere regelmäßige, Abfrage des externen Schnittstellensystems (16) nach neuen, von dem externen Schnittstellensystem (16) für die lokalen Analysemodule (14) bereitgestellten, Skripten durchzuführen.

4. Analysesystem (36) nach Anspruch 3, **dadurch gekennzeichnet, dass** das Skript-Abholmodul (22) dazu vorgesehen ist, bei einem Auffinden eines neu bereitgestellten Skripts mittels der von den Analyseumgebungen (10, 10') ausgehenden Verbindungen, insbesondere der Outbound-Verbindungen, einen Download des bereitgestellten Skripts von dem externen Schnittstellensystem (16) zu veranlassen.

5. Analysesystem (36) nach einem der vorhergehenden Ansprüche, **dadurch gekennzeichnet, dass** die Analyseumgebung (10, 10') eine lokale Programmbibliothek-Datenbank (24) aufweist, welche Programmbibliotheken und/oder Software-Hilfsmodule umfasst, die von den Skripten aufrufbar sind, **und dass** in der Analyseumgebung (10, 10') ein durch die Skripte initiiertes Aufrufen und/oder Herunterladen von externen Programmbibliotheken und/oder Software-Hilfsmodulen, die nicht in der lokalen Programmbibliothek-Datenbank (24) umfasst sind, unterbunden ist.

6. Analysesystem (36) nach Anspruch 5, **dadurch gekennzeichnet, dass** die Analyseumgebungen (10, 10') jeweils zumindest ein Bibliothek-Abholmodul (26) umfassen, welches dazu vorgesehen ist, eine, insbesondere regelmäßige, Abfrage des externen Schnittstellensystems (16) nach neuen, von dem externen Schnittstellensystem (16) für die lokale Programmbibliothek-Datenbank (24) bereitgestellten, insbesondere autorisierten, externen Programmbibliotheken und/oder Software-Hilfsmodulen durchzuführen und insbesondere bei einem Auffinden einer/eines neu bereitgestellten, insbesondere autorisierten, externen Programmbibliothek und/oder Software-Hilfsmoduls mittels der von den Analyseumgebungen (10, 10') ausgehenden Verbindungen, vorzugsweise der Outbound-Verbindungen, einen Download der/des bereitgestellten Programmbibliothek und/oder Software-Hilfsmoduls, vorzugsweise von dem externen Schnittstellensystem (16), zu veranlassen.

7. Analysesystem (36) nach Anspruch 5 oder 6, **dadurch gekennzeichnet, dass** das externe Schnittstellensystem (16) eine Skript-Testumgebung aufweist, welche dazu vorgesehen ist, neue Skripte oder sich in der Entwicklung befindliche Skripte vor einer Bereitstellung zur Übergabe an die Analyseumgebung/en (10, 10'), insbesondere anhand von durch das externe Schnittstellensystem (16) bereitgestellten künstlichen Testumgebungs-Patientendaten, zu testen.

8. Analysesystem (36) nach einem der vorhergehenden Ansprüche, **dadurch gekennzeichnet, dass** die von dem lokalen Analysemodul (14) ausführbaren Skripte, Analyse-Skripte zur rein algorithmischen, insbesondere ML-unabhängigen, Auswertung der Teile der föderierten Patientendaten, auf die das lokale Analysemodul (14) Zugriff hat, sein können.

9. Analysesystem (36) nach einem der vorhergehenden Ansprüche, **dadurch gekennzeichnet, dass** die von dem lokalen Analysemodul (14) ausführbaren Skripte, Skripte zur Erzeugung von Modellen für maschinelles Lernen sein können, die insbesondere zur auf maschinellem Lernen basierten Auswertung der Teile der föderierten Patientendaten, auf die das lokale Analysemodul (14) Zugriff hat, vorgesehen sind.

10. Analysesystem (36) nach einem der vorhergehenden Ansprüche, **dadurch gekennzeichnet, dass** die Analyseumgebungen (10, 10') jeweils zumindest ein Ausgabemodul (28) umfassen, welches zu einer Ausgabe von Analyseergebnissen der lokalen Analysemodule (14) vorgesehen ist, wobei jegliche Ausgabe von Analyseergebnissen nach extern, z.B. an das externe Schnittstellensystem (16), auf eine Ausgabe an, z.B. in einer Whitelist, vorab festgelegte externe Server (30) beschränkt ist.

11. Analysesystem (36) nach einem der vorhergehenden Ansprüche, **dadurch gekennzeichnet, dass** die Analyseumgebungen (10, 10') jeweils zumindest ein Ausgabemodul (28) umfassen, welches zu einer Ausgabe von Analyseergebnissen der lokalen Analysemodule (14) vorgesehen ist, wobei jegliche Ausgabe von Analyseergebnissen nach extern, z.B. an das externe Schnittstellensystem (16), auf aggregierte Daten beschränkt ist.

12. Analysesystem (36) nach Anspruch 9, **dadurch gekennzeichnet, dass** die Modelle für maschinelles Lernen auf das jeweilige lokale Analysemodul (14) beschränkt sind.

13. Analysesystem (36) nach einem der vorhergehenden Ansprüche, **gekennzeichnet durch** lokale Datenextraktionsmodule (32), welche dazu vorgesehen sind, Patientenakten eines Gesundheitsinformationssystems (20, 20'), insbesondere eines Spitalinformationssystems auszulesen und daraus den jeweils der Analyseumgebung (10, 10') zugeordneten Teil der föderierten Patientendaten zu erstellen, auf den die jeweiligen lokalen Analysemodule (14) Zugriff besitzen.

14. Analysesystem (36) nach einem der vorhergehenden Ansprüche, **dadurch gekennzeichnet, dass** die Analyseumgebungen (10, 10') jeweils eine lokale Benutzerschnittstelle (34) umfassen, welche eine On-Site-Analyse des lokal vorhandenen Teils der föderierten Patientendaten erlaubt und/oder welche eine On-Site-Ausführung der Skripte auf den lokal vorhandenen Teil der föderierten Patientendaten ermöglicht.

15. Analyseumgebung (10, 10') oder Anwender- und/oder Programmier-Schnittstellensystem (16) eines Analysesystems (36) nach einem der vorhergehenden Ansprüche.

16. Datenschutzwahrendes Analyseverfahren für föderierte Patientendaten mittels eines Analysesystems (36) nach einem der Ansprüche 1 bis 14, wobei über das externe Anwender- und/oder Programmier-Schnittstellensystem (16) die von den lokalen Analysemodulen (14) ausführbaren Skripte erstellt und/oder bereitgestellt werden und wobei Daten und Skripte zwischen den lokalen Analysemodulen (14) und dem externen Schnittstellensystem (16) ausschließlich über von der jeweiligen Analyseumgebung (10, 10') ausgehende Verbindungen, insbesondere Outbound-Verbindungen, transferiert werden.
